# EUROPÄISCHE PATENTSCHRIFT

(11) **EP 4 205 707 B1**
(45) Veröffentlichungstag und Bekanntmachung des Hinweises auf die Patenterteilung: **01.04.2026**
(21) Anmeldenummer: 22216210.9
(22) Anmeldetag: 22.12.2022
(51) Int. Cl.: A61F 5/01, A63B 23/16, A63B 21/00, A63B 21/045, A63B 21/055, A61F 5/058

(54) **MODIFIZIERBARE UNIVERSALSCHIENE ZUR HANDGELENK- UND FINGERSTABILISIERUNG UND MODULARER BAUSATZ ZU DEREN HERSTELLUNG**
MODIFIABLE UNIVERSAL SPLINT FOR WRIST AND FINGER STABILIZATION, AND MODULAR KIT FOR PRODUCING SAME
ATTELLE UNIVERSELLE MODIFIABLE POUR STABILISATION DU POIGNET ET DU DOIGT ET KIT MODULAIRE POUR SA FABRICATION

(30) Priorität: 30.12.2021 DE 102021006428
(43) Veröffentlichungstag der Anmeldung: 05.07.2023
(73) Patentinhaber: Wienert, Peter, 83483 Bischofswiesen (DE)
(72) Erfinder: Wienert, Peter, 83483 Bischofswiesen (DE); Kolb, Uwe, 83471 Schönau am Königssee (DE); Keilwerth, Helmuth, 83471 Schönau am Königssee (DE)
(74) Vertreter: Banse & Steglich Patentanwälte PartmbB

(56) Entgegenhaltungen:
- EP-B1- 2 991 595
- DE-U1- 202015 008 342
- SE-C2- 539 290
- US-A- 5 490 409
- US-A1- 2006 211 964

## Beschreibung

Die Erfindung betrifft Handschienen, die zur Handgelenk- oder Fingerstabilisierung eines Trägers beispielsweise bei Überlastungssyndromen oder zu konservativen Therapien nach einer Prellung, Knochenfraktur und dergleichen einsetzbar sind. Dabei richtet sich die Erfindung insbesondere auf in ihrer Gestalt modifizierbare Schienen, die an unterschiedliche Anatomien und Krankheitsbilder der Träger anpassbar und wiederverwendbar sind.

Herkömmliche Schienen, ob vorgefertigt oder direkt anatomisch angelegt (beispielsweise als ein Gips- oder ein Castverband), sind in aller Regel nicht wiederverwendbar, weil sie sehr verletzungstypisch gefertigt und/oder nach der Benutzung unhygienisch sind. Damit sind solche Schienen sowohl aus ökonomischer als auch aus ökologischer Sicht nicht mehr zeitgemäß.

Im Stand der Technik sind einige wenige Lösungsansätze für modular zusammensetzbare Handschienen bekannt, die dadurch nachträglich in gewissem Umfang an eine individuelle Anatomie des Trägers anpassbar sind. So offenbart beispielsweise DE 44 38 568 A1 eine Streckschienenvorrichtung, die als (begleitendes) Hilfsmittel für im Anschluss an eine Operation durchzuführende gymnastische Übung verwendet werden kann oder derartige Übungen völlig ersetzt, aber auch zur (vorübergehenden) Ruhigstellung von Gliedmaßen verwendet werden kann. Die Schienenvorrichtung gemäß DE 44 38 568 A1 ist zur Befestigung im Bereich des Handgelenks sowie im Fingerbereich des Trägers ausgebildet und umfasst hierzu einen den Handrücken des Trägers im befestigten Zustand übergreifenden Schienenbereich, eine mit dem Schienenbereich verbundene erste Befestigungsvorrichtung zur Befestigung der Schienenvorrichtung im Bereich des Handgelenks sowie eine mit dem Schienenbereich verbundene zweite Befestigungsvorrichtung zur Befestigung der Schienenvorrichtung im Bereich der Finger des Trägers. Dabei besteht die erste Befestigungsvorrichtung aus lösbaren und wiederverschließbaren, den Unterarm des Trägers im Handgelenkbereich umfassenden Verschlussmitteln, die insbesondere als Klettverschlussbereiche ausgebildet sein können. Die zweite Befestigungsvorrichtung ist durch mindestens ein Steckteil mit mindestens einer Stecköffnung zur Aufnahme und Herausnahme mindestens eines Fingers des Trägers der Schienenvorrichtung ausgebildet, wobei in die Stecköffnungen zur Anpassung an die individuelle Fingeranatomie unterschiedliche Einsatzteile eingeschoben werden können.

Insbesondere kann die Schienenvorrichtung gemäß DE 44 38 568 A1 aus einzelnen, den Schienenbereich, die Steckteile mit Stecköffnungen und die Verschlussmittel bildenden Bauelementen modular aufgebaut sein. Dadurch können abhängig vom medizinischen Behandlungszweck und von der Handanatomie des Trägers beispielsweise ein oder mehrere Steckteile auf verschiedene Art und Weise auf dem Schienenbereich befestigt werden.

Ferner schlägt beispielsweise DE 20 2013 000 878 U1 ein Fingerstützmodul und eine modular aufgebaute Handstützeinrichtung mit einem derartigen Fingerstützmodul und einer Handgelenkorthese vor. Die Handgelenkorthese dient dabei der Stabilisierung des Handgelenks eines Patienten und der Einschränkung seines Bewegungsbereichs, wobei das Fingerstützmodul zusätzlich die Möglichkeit bietet, auch den Fingerbereich einer Hand therapiegerecht zu lagern. Hierzu umfasst das Fingerstützmodul einen Schienenabschnitt, der zum Anlegen an einer Handgelenkorthese ausgebildet ist, eine am Schienenabschnitt vorgesehene Befestigungseinrichtung zur lösbaren Befestigung des Fingerstützmoduls an der Handgelenkorthese sowie einen sich an den Schienenabschnitt anschließenden Fingerauflageabschnitt, der eine Fingerauflagefläche aufweist.

Die Druckschrift DE 20 2015 008342 U1 offenbart eine Handorthese mit einer Unterarmspange und einer über ein Verbindungsstück mit der Unterarmspange verbundenen Mittelhandspange, wobei die Handorthese weiter mindestens ein an der Mittelhandspange befestigtes im Wesentlichen in distal-proximaler Richtung verlaufendes biegeelastisches Element und je ein im Bereich des distalen Endes des biegeelastischen Elements vorgesehenes Befestigungselement zur Befestigung an einem Finger aufweist.

Die Druckschrift US 2006/211964 A1 offenbart eine explizit zur dorsalen Verwendung ausgebildete Handschiene, bei der ein Unterarm-Unterstützungsabschnitt integral (d. h. untrennbar) mit einem Hand-Unterstützungsabschnitt verbunden ist. Die Schiene umfasst ferner lösbar daran und an den Fingern befestigte federnde Streben für je einen Finger.

Die Druckschrift SE 539290 C2 offenbart eine Handgelenk-Orthese, umfassend: eine Schiene mit einem stützenden Schienenabschnitt, der so angepasst ist, dass er sich über die Gelenke (MCP-Gelenke) der Finger der Hand eines menschlichen Patienten erstreckt, wobei der stützende Schienenabschnitt eine dorsale Kopffläche aufweist, die so angepasst ist, dass sie zur Handfläche der Hand gerichtet ist; und mit einem Handgelenk- und Unterarmschienenabschnitt, der mit dem Stützschienenabschnitt integriert oder verbunden ist, wobei sich der Handgelenk- und Unterarmschienenabschnitt von dem Stützschienenabschnitt bis zu einem Abstand erstreckt, der mindestens einem Teil des Unterarms des menschlichen Patienten entspricht. Es ist eine erste aufblasbare Blase vorgesehen, die an der dorsalen Kopffläche des Stützschienenabschnitts an einem MCP-Abschnitt des Stützschienenabschnitts angeordnet ist, um mit den MCP-Gelenken der Hand ausgerichtet zu sein. Der stützende Schienenabschnitt ist so angepasst, dass er sich über mindestens einen größeren Teil der Finger erstreckt. Die Handgelenk-Orthese umfasst eine zweite aufblasbare Blase, die auf der dorsalen Hauptfläche des stützenden Schienenabschnitts an einem Phalanxabschnitt des stützenden Schienenabschnitts angeordnet ist.

Die Druckschrift EP 2 991 595 B1 offenbart eine orthopädische Vorrichtung, umfassend: einen Oberarmabschnitt zur Aufnahme eines oberen Teils eines Arms einer Person; einen Unterarmabschnitt zur Aufnahme eines Unterarmteils des Arms; und mindestens ein Ellbogengelenk, das den Oberarmabschnitt und den Unterarmabschnitt drehbar verbindet, wobei der Unterarmabschnitt eine Freigabesteuerung umfasst, die sich an einem distalen Ende des Unterarmabschnitts befindet und funktionell mit dem mindestens einen Ellbogengelenk gekoppelt ist, wobei die Freigabesteuerung so konfiguriert ist, dass sie das mindestens eine Ellbogengelenk von einem eingeschränkten Bewegungszustand in einen freien Bewegungszustand überführt, wenn die Freigabesteuerung aktiviert wird.

Die Druckschrift US 5 490 409 A offenbart eine einstellbare Stangenbiegevorrichtung mit Nockenwirkung zum Biegen einer chirurgischen Stange, die zwei Griffe umfasst, die um einen Drehzapfen biegbar sind, wobei der Drehzapfen einen drehbaren exzentrischen Biegeknopf aufweist, der auf dem Drehzapfen montiert ist, wobei der Biegeknopf in eine von mehreren ausgewählten Radiusbiegepositionen für den Gebrauch drehbar ist, um eine chirurgische Stange in eine Aussparung zwischen dem drehbaren Biegeknopf und einem Paar von Drehpunkten einzuführen und zu biegen, wobei jeder Drehpunkt an einem oberen Abschnitt jedes der Griffe angeordnet ist, wobei der Stab durch Kontakt jedes der Drehpunkte mit dem Stab biegbar ist, wobei die Drehpunkte den Stab innerhalb der Aussparung mit dem Biegeknopf in Kontakt bringen, wobei der Biegeknopf in eine Vielzahl von abwechselnden auswählbaren Positionen der Radiusbiegung drehbar ist, wobei der Stabbieger ferner einen Kniehebel aufweist, der den Biegeknopf festhält, während die Griffe bewegt werden, wobei der Kniehebel zwei bewegliche Arme aufweist, die an einem weiteren Drehpunkt durch einen Stift verbunden sind, wobei der Stift in einem ausgewählten Kanal einer Vielzahl von Kanälen, die sich auf einer Rückseite einer Platte befinden, verschiebbar ist, wobei die Platte zwischen den beiden Griffen und dem Biegeknopf positioniert ist, gegen den der Biegeknopf drehbar in eine der abwechselnden Positionen der Radiusbiegung bewegbar ist, wobei der Stift gleitend in jedem der Kanäle bewegbar ist, wobei der Biegeknopf in eine gewünschte Verriegelungsposition gegen die runde Platte drehbar ist. Es ist Aufgabe der vorliegenden Erfindung, eine alternative und insbesondere im Hinblick auf die Einfachheit und zugleich Leistungsfähigkeit bei der Handhabung und Herstellung verbesserte Schiene zur Handgelenk- und Fingerstabilisierung eines Trägers bereitzustellen, die für möglichst viele verschiedene Krankheitsbilder präzise anpassbar ist.

Diese Aufgabe wird durch eine modifizierbare Universalschiene gemäß Anspruch 1 sowie durch einen modularen Bausatz zu deren Herstellung oder Anpassung gemäß dem nebengeordneten Anspruch gelöst. Weitere Ausgestaltungen sind in den abhängigen Ansprüchen angegeben. Alle in den Ansprüchen und der nachfolgen Beschreibung für die Universalschiene genannten weiterführenden Merkmale und Wirkungen gelten auch in Bezug auf den modularen Bausatz, wie auch umgekehrt.

Gemäß einem ersten Aspekt ist eine modifizierbare Universalschiene vorgesehen, die zur Handgelenk- und/oder Fingerstabilisierung ihres Trägers bzw. Patienten ausgebildet ist. Die hierin vorgestellte Universalschiene ist durch ihre Modifizierbarkeit sowohl für unterschiedliche Anwendungsfälle und Krankheitsbilder als auch für unterschiedliche Handanatomien einzelner Träger individualisierbar und wiederverwendbar. (Die Universalschiene kann dabei - muss jedoch nicht zwingend - zudem so ausgestaltet sein, dass sie sowohl für die linke als auch für die rechte Hand universell verwendbar ist.)

Hierzu umfasst die Universalschiene erstens eine zum Abstützen einer Handfläche (d. h. einer inneren Fläche bzw. einer Unterseite der Hand ohne Finger) starr ausgebildete flächige Basisplatte, auf der die Handfläche des Trägers beim Tragen der Universalschiene idealerweise fast mit ihrer/seiner gesamten Fläche aufliegen kann. An der Basisplatte ist dauerhaft oder lösbar mindestens ein flexibles und in Bezug auf seinen Umfang individuell anpassbares Handbefestigungsband befestigt, das zum Umlegen um die Hand des Trägers zur Fixierung der Basisplatte daran ausgebildet ist. Die Basisplatte kann beispielsweise aus Metall oder einem anderen im relevanten Kraftbelastungsbereich starren Werkstoff wie Kunststoff etc. mit einer für die Stabilität der Basisplatte geeignet gewählten Dicke gefertigt sein.

Ferner umfasst die Universalschiene eine lösbar an der Basisplatte befestigbare (oder befestigte), zum Abstützen eines Unterarms starr ausgebildete längliche Unterarmschiene. An der Unterarmschiene ist dauerhaft oder lösbar mindestens ein flexibles und in Bezug auf seinen Umfang individuell anpassbares Unterarmbefestigungsband befestigt, das zum Umlegen um den Unterarm eines Trägers zur Fixierung der Unterarmschiene daran ausgebildet ist.

Des Weiteren umfasst die Universalschiene mindestens ein lösbar an der Basisplatte befestigbares (oder befestigtes) Fingerelement. Es können je nach Anwendungsfall und erforderlicher Stabilisierung eines oder mehrerer Finger des Trägers der Universalschiene ein, zwei, drei, vier oder fünf Fingerelemente an der Basisplatte befestigt sein. Jedes Fingerelement umfasst eine zum Abstützen jeweils eines ganzen Fingers länglich ausgebildete Fingerschiene und mindestens ein daran permanent oder abnehmbar befestigtes flexibles und in Bezug auf seinen Umfang individuell anpassbares Fingerbefestigungsband, das zum Umlegen um den Finger des Trägers zur Fixierung der Fingerschiene daran ausgebildet ist.

Die Universalschiene kann bei spezifischen Anwendungsfällen, die keine Fingerstabilisierung erfordern und bei denen beispielsweise eine Stabilisierung bzw. Ruhigstellung eines Handgelenks durch die Kombination der Basisplatte mit der Unterarmschiene ausreichend ist, auch gänzlich ohne Fingerelemente eingesetzt werden. Umgekehrt ist aber auch der Einsatz einzelner oder mehrerer Fingerelemente der Universalschiene mit oder ohne die Basisplatte und gänzlich ohne die Unterarmschiene möglich, wenn die Fingerstabilisierung bzw. - Ruhigstellung im Vordergrund steht.

Dabei ist jede Fingerschiene für transversale (d. h. quer zu deren Längsrichtung wirkende) Kraftbelastungen in einem ersten Kraftintervall, das mit einem menschlichen Finger aufbringbare Kräfte abdeckt, starr oder nur in einem vorbestimmten, zur stabilisierenden Wirkung geeigneten Ausmaß elastisch biegsam ausgebildet. Dabei ist die Fingerschiene in einem höheren (d. h. insgesamt deutlich über dem ersten Kraftintervall liegenden) zweiten Kraftintervall derart plastisch verformbar, dass sie im Wege einer plastischen Verbiegung durch Kräfte im zweiten Kraftintervall dauerhaft an eine individuelle Fingerform und -Ausrichtung eines Trägers der Universalschiene anpassbar ist.

Die Universalschiene ist dabei insbesondere so ausgebildet (vgl. die beigefügten Figuren), dass sie beim Tragen volar (d. h. handinnenflächenseitig) an der Handfläche und entsprechend auch an einer Unterseite des Unterarms bzw. des jeweiligen Fingers des Trägers anliegt. Alternativ und nicht erfindungsgemäß kann die Universalschiene aber auch zu einer dorsalen Nutzung (d. h. oberseitig der Hand, des Unterarms und der Finger) ausgelegt sein.

Mit dieser Ausgestaltung der Universalschiene kann sowohl eine einfache Handhabbarkeit bei der Individualisierung, beim Anlegen und Tragen der Schiene als auch eine für jede Indikation ausreichende Stabilität im gesamten Unterarm- und Handbereich bei notwendiger elastischer Flexibilität gewährleistet werden. Für die jeweilige Funktionalität der einzelnen Bestandteile der Universalschiene geeignete Werkstoffe sind dabei sinnvollerweise so zu wählen, dass sie auch bei mehrmaliger Benutzung wiederverwendbar bleiben, d. h. insbesondere sterilisierbar und/oder waschbar sind. Geeignet können in diesem Sinne beispielsweise unschädliche Metalle und Metalllegierungen, recyclebare Kunststoffe, Befestigungsbänder und Überzüge mit Polstern aus waschbaren Stoffen (beispielsweise aus Textilien) und vieles mehr sein. Je nach Einsatzgebiet, Sterilisierungsmethode und Anwendungsfall kann es zudem sinnvoll sein, das sämtliche Materialien der Universalschiene in einem gesamten Temperaturbereich von beispielsweise etwa -30° C bis etwa +50° C haltbar sind und/oder sogar voll funktionsfähig bleiben. Insbesondere bei der Anwendung im medizinischen Bereich sind mit der vorliegenden Universalschiene aufgrund ihres schlichten und modularen Aufbaus notwendige Hygienestandards einhaltbar. Insbesondere kann die gesamte Universalschiene oder zumindest deren mit dem Körper des Trägers kontaktierende Bestandteile (wie textiler Überzug, siehe unten) auch für eine Waschmaschinenwäsche bei beispielsweise 60° C oder noch höherer Waschtemperatur ausgelegt sein. Insbesondere kann die Schiene oder deren einzelne Bestandteile für eine regelmäßig Sterilisierung durch die Anwendung hoher Temperaturen im Bereich von beispielsweise bis +60° C, bis +70° C oder noch höher ausgelegt sein. Für starr ausgebildete Schienen und Platten kann zudem eine bakterielle Desinfektion in einem noch höheren Temperaturbereich oder mit anderen chemischen Mitteln möglich sein.

Die Universalschiene der hierin dargelegten Art zeichnet sich zudem durch ein kompaktes und in der Praxis handliches Design aus, das nicht nur einfach, sondern auch praktisch überall einsetzbar ist. Möglicher medizinischer oder therapeutischer Indikationsbereich der Universalschiene kann insbesondere - jedoch nicht beschränkend - Synovitiden (Gelenkinnenhautentzündungen), periphere Infektionen, Überlastungssyndrome, aber auch konservative Therapien von beispielsweise Prellungen, Distorsionen, nicht dislozierten Frakturen, Schwellungen, ferner auch CTS (Deutsch: Karpaltunnelsyndrom, KTS), Nachbehandlung von operativ behandelten Frakturen und vieles mehr umfassen.

Die Universalschiene kann aufgrund von deren einfacher Handhabung, Anpassbarkeit und Wiederverwendbarkeit in sehr verschiedenen Einrichtungen und Situationen zum Einsatz kommen, die unter Umständen sehr schnelle, vielfältige und zugleich zuverlässige Hilfeleistungen/Lösungen mit vergleichsweise geringem Aufwand, oder aber auch immer wiederkehrende hochqualifizierte medizinische und therapeutische Maßnahmen erfordern. Hierzu zählen daher beispielsweise alle Arztpraxen, die Handschienen sofort oder für längere bzw. wiederkehrende Behandlung der Handpartie benötigen, wie beispielsweise Allgemeinmediziner, Orthopäden, Chirurgen, Neurologen etc. Weitere Einsatzgebiete sind beispielsweise Behandlungen von Verletzungen oder als vorbeugende Schutzmaßnahmen für Rettungsdiensteinsätze, Sanitäter, bei Soldaten, Sportlern (wie z. B. Kletterern), bei Katastropheneinsätzen, in Altenheimen, aber auch für den Einsatz durch Krankenkassen und Berufsgenossenschaften.

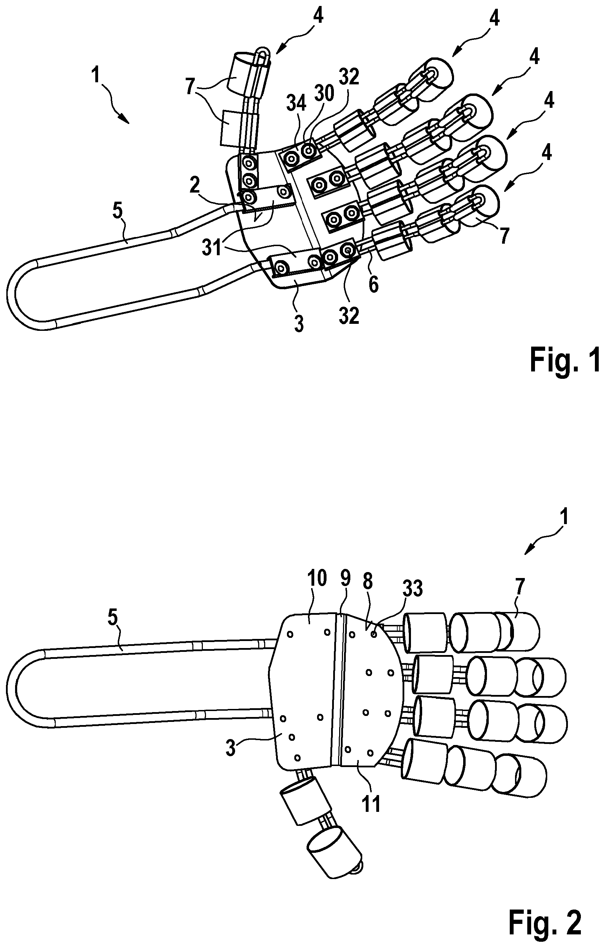

Gemäß einer ersten Ausführungsform (vgl. die beigefügten Figuren) ist jede Fingerschiene aus einem in etwa dreieckig, rechteckig oder U-förmig zu einem Doppeldraht gebogenen Draht gebildet, dessen Drahtstärke den für die Fingerschiene genannten Kraftintervallen entspricht. Als Drahtmaterial eignen sich daher insbesondere Metalle bzw. Metalllegierungen, aber auch beliebige andere Werkstoffe mit vergleichbaren Materialeigenschaften. Metalle können sich auch im Hinblick auf die Sterilisierbarkeit der Schiene durch hohe Temperaturen, etwa im kochenden Wasser oder Dampf oder auch höher, besonders gut eignen. Dabei ist der U-förmige Doppeldraht mit den Enden seiner beiden U-Schenkel an der Basisplatte befestigt (vgl. Fig. 1-4), sodass sein U-förmiges Ende einen freien distalen Längenabschnitt der Fingerschiene bildet.

Alternativ zu dieser in den beigefügten Figuren gezeigten Ausgestaltungsvariante kann eine Fingerschiene bei dieser ersten Ausführungsform auch als ein Doppeldraht ausgebildet sein, der aus zwei separaten und miteinander verbundenen Einzeldrähten besteht. Die beiden Einzeldrähte können beispielsweise an deren an der Basisplatte befestigten proximalen Enden und zusätzlich an deren entgegengesetzten, d. h. distalen Enden und/oder in anderen Längenabschnitten der jeweiligen Fingerschiene miteinander verbunden sein (beispielsweise durch geeignete Querstreben, die mit den beiden Einzeldrähten verklebt, verschweißt oder verschraubt sein können).

Bei dieser ersten Ausführungsform können die beiden Schenkel bzw. Einzeldrähte des Doppeldrahts insbesondere etwa parallel zueinander in einem Abstand von etwas weniger als einer Fingerbreite (was beispielsweise etwa 1 cm entsprechen kann) voneinander verlaufen, derart dass sie bei der bestimmungsgemäßen Verwendung der Universalschiene einen auf der Fingerschiene aufliegenden Finger des Trägers entlang seiner Längsachse zu beiden Seiten davon abstützen. Alternativ oder zusätzlich kann der Doppeldraht an einer oder mehreren entlang seiner Länge verteilten Stellen zur Anpassung an eine individuelle Fingerform und -Ausrichtung in einer transversalen Richtung, die senkrecht zu einer von den beiden Schenkeln/Einzeldrähten jeweils lokal aufgespannten Ebene steht, plastisch verbogen sein/werden. Diese Übergangsstellen sind dabei entlang der Fingerschiene sinnvollerweise an Stellen auszubilden, die den Fingergelenken des jeweiligen Trägers entsprechen. An diesen Übergangsstellen kann eine Fingerschiene zur Anpassung an die individuelle Fingerstellung- bzw. Krümmung des Trägers durch plastische Biegung individuell geformte Anstellwinkel von beispielsweise bis zu 90° aufweisen.

Alternativ zu der ersten Ausführungsform kann eine Fingerschiene aber auch als ein einfacher Einzeldraht einer geeigneten Drahtstärke oder als ein flächiges, beispielsweise rechteckiges oder auch in Endabschnitten abgerundetes, Band aus einem Material geeigneter Stärke/Dicke ausgebildet sein. Beispielsweise kann die hierin beschriebene Funktionalität mit einem etwa 1 cm breiten und wenige Millimeter dicken Metallband erzielt werden.

Alternativ oder zusätzlich zu einer in Längsrichtung der Fingerschiene ununterbrochenen Ausgestaltung kann eine Fingerschiene beispielsweise auch modular aus mehreren durch Steckverbindungen, Gelenkverbindungen (beispielsweise über mindestens ein Kugel-Presspassgelenk) oder dergleichen miteinander lösbar verbunden Längenabschnitten aufgebaut sein, die von Anwendungsfall zu Anwendungsfall unterschiedlich wählbar und kombinierbar sind. Die Steck- oder Gelenkverbindungen sind dabei entlang der Fingerschiene sinnvollerweise an Stellen angeordnet, die den Fingergelenken entsprechen. An diesen Übergangsstellen kann eine Fingerschiene zur Anpassung an die individuelle Fingerstellung- bzw. Krümmung des Trägers einen Winkelstellungsfreiheitsgrad von beispielsweise bis zu 90° aufweisen.

Bei einer spezifischen Ausgestaltung ist auch die Unterarmschiene für transversale (d. h. quer zu deren Längsrichtung wirkende) Kraftbelastungen in einem dritten Kraftintervall, das mit einem menschlichen Unterarm beim Tragen der Universalschiene darauf aufbringbare Kräfte abdeckt, starr oder in vorbestimmten Grenzen elastisch biegsam, während sie in einem höheren (d. h. insgesamt deutlich über dem dritten Kraftintervall liegenden) vierten Kraftintervall derart plastisch verformbar ist, dass sie durch eine plastische Verbiegung durch Kräfte im vierten Kraftintervall dauerhaft an eine individuelle Unterarmform und - Ausrichtung anpassbar ist. Dadurch kann auch die Unterarmschiene durch plastische Verformung an eine individuelle Anatomie und Indikation präzise angepasst werden.

Gemäß einer zweiten Ausführungsform, die insbesondere mit der obigen ersten Ausführungsform und deren oben genannten Alternativen vollumfänglich kombinierbar ist, ist auch die Unterarmschiene aus einem in etwa dreieckig, rechteckig oder U-förmig zu einem Doppeldraht gebogenen Draht gebildet, dessen Drahtstärke den für die Unterarmschiene genannten Kraftintervallen entspricht (vgl. Fig. 1-4). Als Drahtmaterial eignen sich auch hier insbesondere Metalle bzw. Metalllegierungen, aber auch beliebige andere Werkstoffe mit vergleichbaren Materialeigenschaften. Metalle können sich wiederum im Hinblick auf die Sterilisierbarkeit der Schiene durch hohe Temperaturen, etwa im kochenden Wasser oder Dampf oder auch höher, besonders gut eignen.

Alternativ zu dieser in den beigefügten Figuren gezeigten Ausgestaltungsvariante kann eine Unterarmschiene bei dieser zweiten Ausführungsform auch als ein Doppeldraht ausgebildet sein, der aus zwei separaten und miteinander verbundenen Einzeldrähten besteht. Die beiden Einzeldrähte können beispielsweise an deren an der Basisplatte befestigten proximalen Enden und zusätzlich an deren entgegengesetzten, d. h. distalen Enden und/oder in anderen Längenabschnitten der Unterarmschiene miteinander verbunden sein (beispielsweise durch geeignete Querstreben, die mit den beiden Einzeldrähten verklebt, verschweißt oder verschraubt sein können).

Bei der genannten zweiten Ausführungsform kann der Doppeldraht der Unterarmschiene insbesondere mit den proximalen Enden seiner beiden Schenkel/Einzeldrähte an der Basisplatte befestigt sein, sodass sein dreieckiges, rechteckiges oder U-förmiges Ende bzw. seine miteinander verbundenen distalen Einzeldrahtenden einen freien distalen Längenabschnitt der Unterarmschiene bildet (vgl. Fig. 1-4) und beispielsweise zum Abstützen des Ellenbogens dienen kann. Die Unterarmschiene kann aber auch deutlich kürzer als die Unterarmlänge eines konkreten Trägers sein, um beispielsweise etwa eine Dreiviertel- oder Halblänge seines Unterarms ab dem Handgelenk abzustützen.

Insbesondere können die beiden Schenkel oder Einzeldrähte des Doppeldrahts der Unterarmschiene etwa parallel zueinander in einem Abstand von etwas weniger als einer Unterarmbreite voneinander verlaufen, derart dass sie einen auf der Unterarmschiene bei deren bestimmungsgemäßer Verwendung aufliegenden Unterarm entlang seiner Längsachse zu beiden Seiten davon abstützen. Alternativ oder zusätzlich kann der Doppeldraht der Unterarmschiene an einer oder mehreren entlang seiner Länge verteilten Stellen zur Anpassung an eine individuelle Unterarmform und/oder Handgelenkstellung in einer transversalen Richtung, die senkrecht zu einer von den beiden Schenkeln/Einzeldrähten der Unterarmschiene jeweils lokal aufgespannten Ebene steht, plastisch verbogen werden bzw. sein.

Alternativ oder zusätzlich zu einem Doppeldraht kann eine Unterarmschiene beispielsweise auch aus einem einfachen Einzeldraht oder aus einem Materialstreifen oder einem Halbrohr einer geeigneten Breite und Materialstärke und/oder modular aus mehreren durch Steckverbindungen oder dergleichen miteinander lösbar verbunden Längenabschnitten aufgebaut sein, die von Anwendungsfall zu Anwendungsfall unterschiedlich gewählt sein können.

Die Basisplatte der hierin vorgestellten Universalschiene weist eine zum Auflegen der Handfläche darauf glatt ausgebildete Oberseite und eine von dieser abgewandte Unterseite, an der die Unterarmschiene und das mindestens eine Fingerelement lösbar befestigt sind, auf. Die Basisplatte kann dabei beispielsweise als eine Drittelrohrauflage ausgebildet sein.

Ferner setzt sich bei der hierin vorgestellten Universalschiene die Oberseite der Basisplatte zusammen aus einem ersten ebenen Flächenabschnitt, der zum Abstützen eines vom Handgelenk etwa bis zu den Fingergrundgelenken reichenden Großteils der Handfläche ausgebildet ist, und einem zweiten ebenen Flächenabschnitt, der zum Abstützen der restlichen Handfläche etwa ab den Fingergrundgelenken ausgebildet ist. Dabei gehen der erste Flächenabschnitt und der zweite Flächenabschnitt ohne Unterbrechung und unter Ausbildung eines geradlinigen (oder alternativ auch gebogenen oder gekrümmten) Knicks in der Oberseite der Basisplatte ineinander über, sodass sie miteinander einen stumpfen Winkel zwischen 180° und 270° bilden (vgl. Fig. 1-4). Der genannte Winkel ist in einer sich von der Oberseite der Basisplatte nach oben erstreckenden normalen Halbebene, die sich rechtwinklig zum Knick erstreckt, zu messen.

Bei einer spezifischen Ausgestaltung können die lösbaren Verbindungen der Unterarmschiene und/oder der Fingerelemente mit der Basisplatte mit Schraubverbindungen ausgebildet sein und die Basisplatte hierzu beispielsweise geeignete Durchgangs- oder Blindlöcher mit oder ohne Innengewinde aufweisen.

Eine solche Schraubverbindung kann eine, zwei oder mehr als zwei Befestigungsschrauben umfassen. Die Befestigungsschrauben können in eines der entsprechenden Durchgangs- oder Blindlöcher in der Basisplatte eingesetzt sein und dort eingeschraubt werden, entweder in ein dort eingeschnittenes Innengewinde oder in eine entsprechende Schraubenmutter. Die Befestigungsschraube(n) können mit einem Klemmelement, z.B. in Form einer Klemmplatte, zusammenwirken, so dass beim Einschrauben der Befestigungsschraube(n) das Klemmelement gegen die Basisplatte gedrückt wird und dadurch das Fingerelement, das mit einem Abschnitt jeweils zwischen dem Klemmelement und der Basisplatte aufgenommen ist, verklemmt und dadurch fixiert.

Vorzugsweise ist die Länge der Befestigungsschrauben so bestimmt, dass nach Festschrauben mit eingesetztem Fingerelement das eingeschraubte Ende der Befestigungsschrauben nicht an der der Schraubverbindung gegenüberliegenden Seite der Basisplatte hervorsteht.

Insbesondere kann dabei eine Längenverstellbarkeit der Unterarmschiene und/oder der Fingerelemente durch deren Verschiebung in deren Längsrichtung entlang der jeweiligen Schraubverbindung in deren teilweise gelöstem Zustand realisiert sein. Eine solche Längenverstellbarkeit ist insbesondere bei der Ausführung der jeweiligen Schiene als Doppeldraht und der Anordnung der Befestigungsschrauben zwischen den beiden Schenkeln bzw. Einzeldrähten des Doppeldrahts in der Regel direkt gegeben. Durch das Vorsehen von mindestens zwei in der Längsrichtung der Fingerelemente angeordneten Befestigungsschrauben für eine betreffende der Schraubverbindungen kann zusätzlich eine Verdrehsicherheit der Fingerelement nach deren Fixieren an der Basisplatte gewährleistet werden, insbesondere indem der Abstand der Befestigungsschrauben größer ist als der Abstand der beiden U-Schenkel des betreffenden Fingerelements.

Ferner kann die Universalschiene zusätzlich eine Ellbogenschiene aufweisen, die zum Abstützen des Ellenbogens und/oder des Oberarms eines Trägers starr und länglich ausgebildet ist und an einem von der Basisplatte abgewandten distalen Ende der Unterarmschiene drehbar mit dieser verbunden ist, derart dass sie aus einem vollständig an der Unterarmschiene angelegten Parkzustand in einen von der Unterarmschiene zu einem geeigneten Anstellwinkel weggeklappten Betriebszustand aufklappbar ist. Dabei umfasst die Universalschiene eine oder mehrere Lösungs- und Arretier-Einrichtungen, die zum Arretieren der Ellbogenschiene an der Unterarmschiene sowohl in deren Parkzustand als auch in deren Betriebszustand in einem geeigneten Anstellwinkel bezüglich der Unterarmschiene und zum Lösen der jeweiligen Arretierung ausgebildet sind. Der geeignete Anstellwinkel kann beispielsweise der gewünschte Ruhestellungswinkel sein, bei dem der Oberarm und der Unterarm mithilfe der Universalschiene relativ zueinander stabilisiert werden sollen. Bei der Kombination mit der obigen zweiten Ausführungsform kann auch die Ellbogenschiene als eine Doppeldraht-Schiene ausgebildet sein. In deren mit der Unterarmschiene zusammengeklapptem Parkzustand können sich beide Drahtschenkel der Ellbogenschiene beispielsweise zu beiden Seiten des Doppeldrahts der Unterarmschiene erstrecken, was eine räumlich besonders kompakte Anordnung für einen Transport oder zur Lagerung der Universalschiene ergibt.

Auch die Ellbogenschiene kann dabei mindestens ein verschließbares flexibles Oberarmbefestigungsband umfassen, das zum Umlegen um den Oberarm zur Fixierung der Ellbogenschiede daran ausgebildet ist.

Bei einer spezifischen Ausgestaltung können die flexiblen Hand-, Unterarm-, Oberarm und/oder Finger-Befestigungsbänder jeweils mit einem Verschluss, beispielsweise mit einem Klettverschluss, verschließbar ausgebildet sein. Der Klettverschluss kann dabei insbesondere derart großflächig ausgebildet sein, dass durch das Variieren des Überlapps der beiden zu verbindenden Bandenden im Klettverschlussbereich der Umfang des Befestigungsbands beim Verschließen variierbar/anpassbar ist. Alternativ zu einer verschließbaren Ausgestaltung kann das jeweilige Band jedoch auch ohne Unterbrechung und damit auch ohne Verschluss ausgebildet sein, sodass der Finger, Unterarm, Oberarm bzw. die Hand des Trägers einfach durch das jeweilige Befestigungsband gesteckt wird. In diesem Fall kann das jeweilige Befestigungsband aus einem in Umfangsrichtung elastisch dehnbaren Material, beispielsweise aus einem Strechgewebe und/oder Gummi, aufgebaut sein, um sowohl eine Anpassbarkeit des Bandumfangs an die individuelle Körpergröße des Trägers als auch eine einfache An- und Ausziehbarkeit beim Anlegen und Abnehmen der Universalschiene zu gewährleisten.

Gemäß einem weiteren Aspekt ist ein modularer Bausatz zum Herstellen bzw. Fertigstellen oder Modifizieren einer Universalschiene der hierin dargelegten Art vorgesehen. Hierzu umfasst der modulare Bausatz unter anderem
- eine oder mehrere unterschiedlich dimensionierte und/oder geformte Basisplatte(n) (gegebenenfalls mit mindestens einem daran permanent oder abnehmbar befestigten verschließbaren flexiblen Handbefestigungsband);
- eine oder mehrere unterschiedlich dimensionierte und/oder geformte, jeweils lösbar an der Basisplatte befestigbare Unterarmschiene(n) (gegebenenfalls mit mindestens einem daran permanent oder abnehmbar befestigten verschließbaren flexiblen Unterarmbefestigungsband); sowie
- für jeden Finger einer Hand mindestens ein oder mehrere unterschiedlich dimensionierte und/oder geformte, jeweils lösbar an der Basisplatte befestigbare Fingerelement(e).

Die genannten Bestandteile des Bausatzes können jeweils paarweise für eine linke und für eine rechte Hand vorliegen und dabei beispielsweise zueinander spiegelsymmetrisch und/oder in anderer Weise unterschiedlich ausgebildet sein. Alle oder einige Bestandteile können aber auch universell zur Anwendung sowohl für die linke als auch für die rechte Hand ausgelegt sein. Ein möglicher Bausatz kann aber auch nur für eine Hand - d. h. nur links oder nur rechts, - vorgesehen sein. Andererseits kann ein für alle Fälle geeigneter kleinster Bausatz zwei Basisplatten, von denen eine für die linke und die andere für die rechte Hand eines Trägers ausgestaltet sind, und einen Satz von fünf Fingerelementen für fünf Finger einer Hand sowie eine einzige Unterarmschiene umfassen.

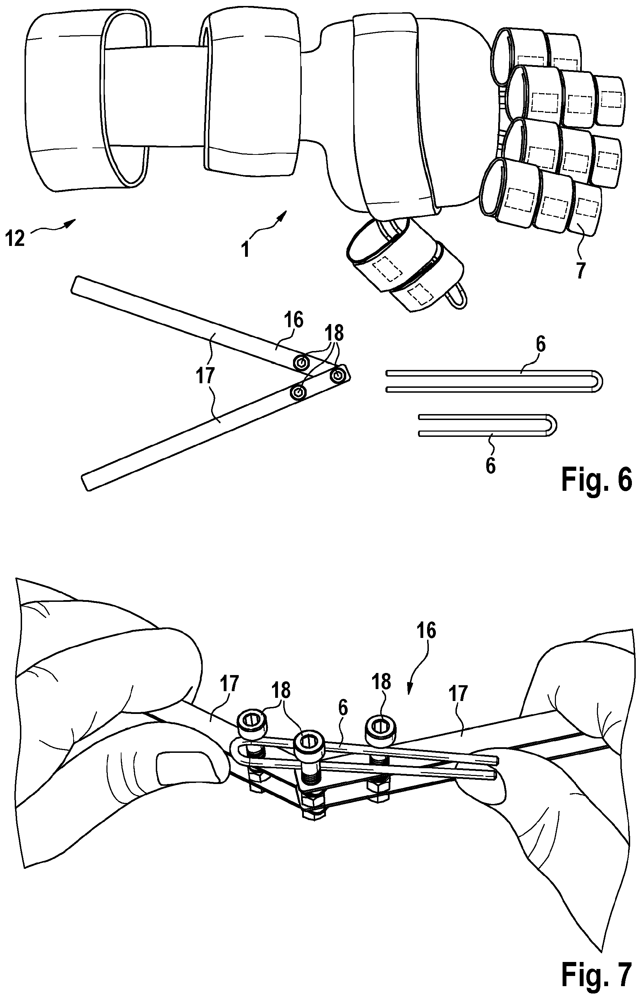

Zum formgebenden plastischen Biegen der Fingerschienen und/oder der Unterarmschiene(n) zu deren Anpassung an unterschiedliche Anwendungsfälle und Trägeranatomien kann der modulare Bausatz ferner ein Schränkeisen als Hilfswerkzeug aufweisen (vgl. Fig. 6 und 7). Das Schränkeisen kann dabei beispielsweise einen Satz von zwei oder mehr drehbar miteinander verbindbaren Konturiereisen mit jeweils einem oder mehreren Vorsprüngen umfassen, die derart ausgebildet sind, dass die zu biegende Finger- oder Unterarmschiene zwischen den Vorsprüngen zweier verschränkter, drehbar miteinander verbundener Konturiereisen einspannbar und durch das Drehen dieser Konturiereisen relativ zueinander zu einer gewünschten Form verbiegbar ist.

Ferner kann der modulare Bausatz einen Überzug aus weichem, insbesondere textilen, Material umfassen, der zum Aufsetzten und hüllenartigen Überziehen der miteinander verbundenen Basisplatte und Unterarmschiene ausgebildet ist.

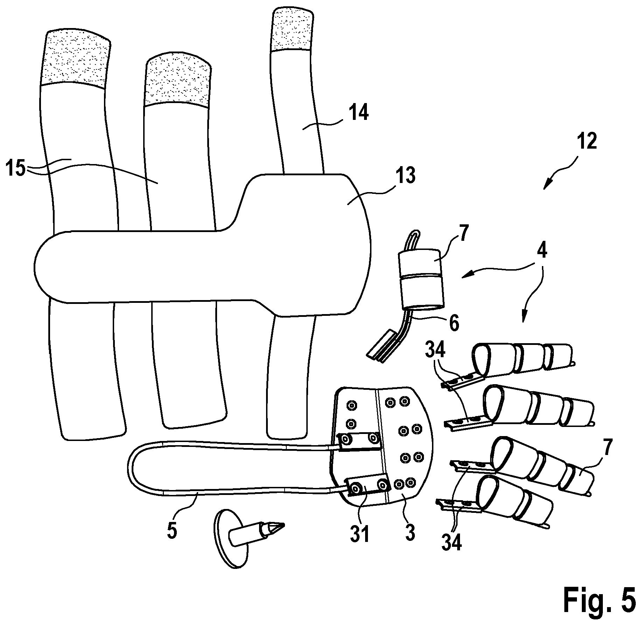

Dabei sind die zugehörigen Hand- und Unterarmbefestigungsbänder im Überzug integriert (vgl. Fig. 5 und 6). Durch den Überzug können die Basisplatte und die Unterarmschiene, die aus starrem Material wie Metall etc. gefertigt sind, weich und damit angenehmer am Körper des Trägers anliegen. Insbesondere können im Überzug zur Vermeidung von Druckstellen am Patientenkörper zusätzlich Polster an geeigneten Stellen integriert sein.

Die obigen Aspekte der Erfindung und deren Ausführungsformen und spezifische Ausgestaltungen werden nachfolgend anhand von in den beigefügten Zeichnungen dargestellten Beispiele näher erläutert. Die Zeichnungen können als maßstabsgetreu oder auch als eine rein schematische Illustration des grundsätzlichen Aufbauprinzips, d. h. nicht als maßstabsgetreu, verstanden werden und anders skalierbar sein als dargestellt. Es zeigen:
- Figur 1: eine perspektivische volare Ansicht eines Ausführungsbeispiels einer modifizierbaren Universalschiene der hierin dargelegten Art in einem zusammengesetzten Zustand ohne Stoffüberzug, mit daran befestigten Fingerelementen für alle fünf Finger eines Trägers und einer daran befestigten Unterarmschiene;
- Figur 2: eine perspektivische dorsale Ansicht der Universalschiene der Fig. 1;
- Figur 3: eine weitere perspektivische Ansicht der Universalschiene der Fig. 1 mit Blick von vorn in Längsrichtung der Unterarmschiene;
- Figur 4: eine Seitenansicht der Universalschiene der Fig. 1;
- Figur 5: einen modularen Bausatz der hierin dargelegten Art für die Universalschiene der Fig. 1 mit separatem Überzug;
- Figur 6: modularer Bausatz der Fig. 5 mit der zusammengesetzten Universalschiene mit aufgesetztem Überzug sowie einem Schränkeisen und zwei weiteren Fingerschienen; und
- Figur 7: ein Beispielschritt zur formgebenden Biegung einer Fingerschiene mit dem Schränkeisen im modularen Bausatz der Fig. 6.

Fig. 1 zeigt eine perspektivische Ansicht eines Ausführungsbeispiels einer modifizierbaren Universalschiene 1 der hierin dargelegten Art in einem zusammengesetzten Zustand mit Blick auf die Unterseite 2 ihrer Basisplatte 3 mit daran durch Schraubverbindungen 30 befestigten Fingerelementen 4 für alle fünf Finger eines Trägers (nicht dargestellt) und einer ebenfalls durch Schraubverbindungen 31 an der Unterseite 2 der Basisplatte 3 befestigten Unterarmschiene 5.

Die hierin dargestellten Ausführungsformen zeigen die Universalschiene 1 für eine linke Hand. Durch gespiegelte Ausführung der Basisplatte 3 (hinsichtlich der Durchgangs- oder Blindlöcher) kann der nachfolgend beschriebene Aufbau auch für eine rechte Hand realisiert werden.

Eine solche Schraubverbindung 30,31 kann eine, zwei oder mehr als zwei Befestigungsschrauben 32 umfassen. Die Befestigungsschrauben 32 können in eines der entsprechenden Durchgangs- oder Blindlöcher 33 in der Basisplatte 3 eingesetzt sein und dort eingeschraubt werden, entweder in ein dort eingeschnittenes Innengewinde oder in eine entsprechende Schraubenmutter. Die Befestigungsschraube(n) 32 können mit einem Klemmelement 34, z.B. in Form einer Klemmplatte, mit durchgängigen Schraubenöffnungen zur Aufnahme der entsprechenden Befestigungsschraube 32, zusammenwirken, so dass beim Einschrauben der Befestigungsschraube(n) 32 das Klemmelement 34 gegen die Basisplatte 3 gedrückt wird und dadurch das Fingerelement 4, das mit einem Abschnitt jeweils zwischen dem Klemmelement 34 und der Basisplatte 3 aufgenommen ist, verklemmt und dadurch an der Basisplatte 3 fixiert.

Jedes Fingerelement 4 umfasst eine zum Abstützen jeweils eines ganzen Fingers länglich ausgebildete Fingerschiene 6 und je zwei bzw. drei elastisch dehnbare und/oder verschließbare flexible Fingerbefestigungsbänder 7, die zum Umlegen um einen Finger zur Fixierung der Fingerschiene 6 daran ausgebildet sind. Die Fingerbefestigungsbänder 7 können beispielsweise jeweils durch Klettverschluss gemäß Fig. 5 und 6 verschließbar sein, der in Fig. 1 nicht dargestellt ist.

**In** diesem Beispiel sind sowohl die Fingerschienen 6 als auch die Unterarmschiene 5 jeweils aus einem U-förmig zu einem Doppeldraht gebogenen Draht einer geeigneten Drahtstärke gebildet, wobei zur Vermeidung von Wiederholungen auf die detaillierte Beschreibung der entsprechenden ersten und zweiten Ausführungsformen hierin weiter oben verwiesen wird.

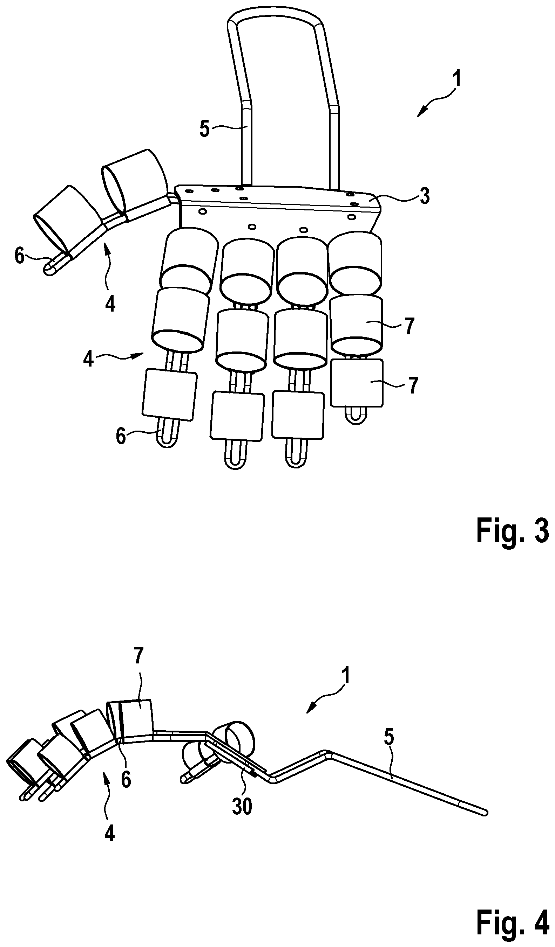

Fig. 2 zeigt eine weitere perspektivische Ansicht der Universalschiene 1 der Fig. 1 mit Blick auf die zum Abstützen einer Handfläche eines Trägers (nicht dargestellt) ausgebildete Oberseite 8 der Basisplatte 3. Wie man besonders gut in dieser und in der weiteren Fig. 4 erkennen kann, ist die Oberseite 8 der Basisplatte 3 in diesem Beispiel mit einem geradlinigen Knick 9 (der alternativ auch gebogen sein kann, nicht dargestellt) zwischen deren erstem ebenen Flächenabschnitt 10 und einem unter einem Winkel zwischen etwa 180° und 270° angewinkelt angeordneten zweiten ebenen Flächenabschnitt 11 ausgebildet, wie hierin weiter oben im allgemeinen Teil der Beschreibungbeschrieben. Zur Vermeidung von Wiederholungen wird an dieser Stelle auf diese detaillierte Beschreibung hierin weiter oben verwiesen.

Fig. 3 zeigt eine weitere perspektivische Ansicht der Universalschiene 1 der Fig. 1 mit Blick von vorn in Längsrichtung der Unterarmschiene 5.

Fig. 4 zeigt eine Seitenansicht der Universalschiene 1, in der die individuell angepassten gebogenen Formen der einzelnen Schienen 6 und 5 besonders gut zu erkennen sind.

Fig. 5 zeigt einen modularen Bausatz 12 der hierin dargelegten Art für die Universalschiene 1 der Fig. 1 mit deren einzelnen Bestandteilen der Fig. 1-4 in einem teilweise auseinandermontierten Zustand sowie mit einem separaten Überzug 13 aus weichem Textilmaterial, der zum Aufsetzten und hüllenartigen Überziehen der miteinander verbundenen Basisplatte 3 und Unterarmschiene 5 ausgebildet ist. Im Überzug 13 sind dabei auch durch Klettverschluss verschließbare Hand- und Unterarmbefestigungsbänder 14 bzw. 15 zur Befestigung der Basisplatte 3 und der Unterarmschiene 5 an der Hand bzw. am Unterarm eines Trägers (nicht dargestellt) integriert.

Fig. 6 zeigt den modularen Bausatz 12 der Fig. 5 mit der vollständig zusammengesetzten Universalschiene 1 und einem zusätzlich vorgesehenen Schränkeisen 16 und zwei weiteren Fingerschienen 6 unterschiedlicher Länge, die damit in die geeignete Form gebogen werden können, wie in Fig. 7 dargestellt. Hierzu umfasst das Schränkeisen 16 in diesem Beispiel zwei drehbar miteinander verbundene Konturiereisen 17 mit jeweils einem bzw. zwei Vorsprüngen 18, zwischen denen die zu biegende Fingerschiene 6 eingespannt ist, um durch das Drehen dieser Konturiereisen 17 relativ zueinander zu einer gewünschten Form verbogen zu werden.

## Patentansprüche

1. Modifizierbare Universalschiene (1) zur Handgelenk- und/oder Fingerstabilisierung, umfassend:
- eine zum Abstützen einer Handfläche starr ausgebildete flächige Basisplatte (3) mit mindestens einem flexiblen und in seinem Umfang individuell anpassbaren Handbefestigungsband (14), das zum Umlegen um die Hand zur Fixierung der Basisplatte (3) daran ausgebildet ist;
- eine lösbar an der Basisplatte (3) befestigbare, zum Abstützen eines Unterarms starr ausgebildete längliche Unterarmschiene (5) mit mindestens einem flexiblen und in seinem Umfang individuell anpassbaren Unterarmbefestigungsband (15), das zum Umlegen um den Unterarm zur Fixierung der Unterarmschiene (5) daran ausgebildet ist; sowie
- mindestens ein lösbar an der Basisplatte (3) befestigbares Fingerelement (4), umfassend eine zum Abstützen jeweils eines ganzen Fingers länglich ausgebildete Fingerschiene (6) und mindestens ein flexibles und in seinem Umfang individuell anpassbares Fingerbefestigungsband (7), das zum Umlegen um den Finger zur Fixierung der Fingerschiene (6) daran ausgebildet ist;
- wobei jede Fingerschiene (6) für transversale Kraftbelastungen in einem ersten Kraftintervall, das mit einem menschlichen Finger aufbringbare Kräfte abdeckt, starr oder in vorbestimmten Grenzen elastisch biegsam ist und in einem höheren zweiten Kraftintervall derart plastisch verformbar ist, dass sie durch eine plastische Biegung durch Kräfte im zweiten Kraftintervall dauerhaft an eine individuelle Fingerform und -Ausrichtung anpassbar ist;
- die Basisplatte (3) eine zum Auflegen der Handfläche darauf glatt ausgebildete Oberseite (8) und eine von dieser abgewandte Unterseite (2), an der die Unterarmschiene (5) und das mindestens eine Fingerelement (4) lösbar befestigt sind, aufweist;
- die Oberseite (8) der Basisplatte (3) sich zusammensetzt aus einem ersten ebenen Flächenabschnitt (10), der zum Abstützen eines etwa bis zu den Fingergrundgelenken reichenden Großteils der Handfläche ausgebildet ist, und einem zweiten ebenen Flächenabschnitt (11), der zum Abstützen der restlichen Handfläche etwa ab den Fingergrundgelenken ausgebildet ist; und
- der erste Flächenabschnitt (10) und der zweite Flächenabschnitt (11) ohne Unterbrechung und unter Ausbildung eines geradlinigen oder gebogenen Knicks (9) in der Oberseite (8) der Basisplatte (3) ineinander übergehen, sodass sie miteinander einen stumpfen Winkel zwischen 180° und 270° bilden, gemessen in einer sich von der Oberseite (8) der Basisplatte (3) nach oben erstreckenden normalen Halbebene, die sich rechtwinklig zum Knick (9) erstreckt.

2. Universalschiene (1) nach Anspruch 1, bei der
- jede Fingerschiene (6) aus einem etwa dreieckig, rechteckig oder U-förmig zu einem Doppeldraht gebogenen Draht gebildet ist oder als ein Doppeldraht aus zwei miteinander verbundenen Einzeldrähten ausgebildet ist;
- wobei die Drahtstärke den genannten Kraftintervallen entspricht und der Doppeldraht mit proximalen Enden seiner beiden Schenkel bzw. Einzeldrähte an der Basisplatte (3) befestigt ist, sodass sein dreieckiges, rechteckiges oder U-förmiges Ende bzw. seine miteinander verbundenen distalen Einzeldrahtenden einen freien distalen Längenabschnitt der Fingerschiene (6) bilden.

3. Universalschiene (1) nach Anspruch 2, bei der
- die beiden Schenkel bzw. Einzeldrähte des Doppeldrahts etwa parallel zueinander in einem Abstand von etwas weniger als einer Fingerbreite voneinander verlaufen, derart dass sie einen auf der Fingerschiene (6) aufliegenden Finger entlang seiner Längsachse zu beiden Seiten davon abstützen; und/oder
- der Doppeldraht an einer oder mehreren entlang seiner Länge verteilten Stellen zur Anpassung an eine individuelle Fingerform und -Ausrichtung in einer transversalen Richtung, die senkrecht zu einer von den beiden Schenkeln bzw. Einzeldrähten jeweils lokal aufgespannten Ebene steht, plastisch verbogen ist.

4. Universalschiene (1) nach einem der vorhergehenden Ansprüche, bei der
- die Unterarmschiene (5) für transversale Kraftbelastungen in einem dritten Kraftintervall, das mit einem menschlichen Unterarm beim Tragen der Universalschiene (5) darauf aufbringbare Kräfte abdeckt, starr oder in vorbestimmten Grenzen elastisch biegsam ist und in einem höheren vierten Kraftintervall derart plastisch verformbar ist, dass sie durch eine plastische Verbiegung durch Kräfte im vierten Kraftintervall dauerhaft an eine individuelle Unterarmform und Handgelenkstellung anpassbar ist.

5. Universalschiene (1) nach Anspruch 4, bei der
- die Unterarmschiene (5) aus einem etwa dreieckig, rechteckig oder U-förmig zu einem Doppeldraht gebogenen Draht gebildet ist oder als ein Doppeldraht aus zwei miteinander verbundenen Einzeldrähten ausgebildet ist;
- wobei die Drahtstärke den für die Unterarmschiene genannten Kraftintervallen entspricht und der Doppeldraht der Unterarmschiene (5) mit proximalen Enden seiner beiden Schenkel bzw. Einzeldrähte an der Basisplatte (3) befestigt ist, sodass sein dreieckiges, rechteckiges oder U-förmiges Ende bzw. seine miteinander verbundenen distalen Einzeldrahtenden einen freien distalen Längenabschnitt der Unterarmschiene (5) bildet.

6. Universalschiene (1) nach Anspruch 5, bei der
- die beiden Schenkel bzw. Einzeldrähte des U-förmigen Doppeldrahts der Unterarmschiene (5) etwa parallel zueinander in einem Abstand von etwas weniger als einer Unterarmbreite voneinander verlaufen, derart dass sie einen auf der Unterarmschiene (5) aufliegenden Unterarm entlang seiner Längsachse zu beiden Seiten davon abstützen; und/oder
- der Doppeldraht der Unterarmschiene (5) an einer oder mehreren entlang seiner Länge verteilten Stellen zur Anpassung an eine individuelle Unterarmform und Handgelenkstellung in einer transversalen Richtung, die senkrecht zu einer von den beiden Schenkeln bzw. Einzeldrähten der Unterarmschiene (5) jeweils lokal aufgespannten Ebene steht, plastisch verbogen ist.

7. Universalschiene (1) nach einem der vorhergehenden Ansprüche, bei der
- die lösbaren Verbindungen der Unterarmschiene (5) und/oder der Fingerelemente (4) mit der Basisplatte als Schraubverbindungen oder Schraub-Klemmverbindungen ausgebildet sind, vorzugsweise mit einer Längenverstellbarkeit der Unterarmschiene (5) und/oder der Fingerelemente (4) durch deren Verschiebung in deren Längsrichtung entlang der jeweiligen Schraubverbindung bzw. Schraub-Klemmverbindungen; und/oder
- die flexiblen Hand-, Unterarm- und/oder Finger-Befestigungsbänder (14, 15, 7) jeweils mit einem Verschluss, insbesondere einem Klettverschluss, verschließbar ausgebildet sind.

8. Universalschiene (1) nach einem der vorhergehenden Ansprüche, ferner umfassend
- eine Ellbogenschiene, die zum Abstützen des Ellenbogens und/oder eines Oberarms starr und länglich ausgebildet ist und an einem von der Basisplatte (3) abgewandten distalen Ende der Unterarmschiene (5) derart drehbar mit dieser verbunden ist, dass sie aus einem vollständig an der Unterarmschiene (5) angelegten Parkzustand in einen von der Unterarmschiene (5) weggeklappten Betriebszustand aufklappbar ist; sowie
- eine oder mehrere Lösungs- und Arretier-Einrichtungen, die zum Arretieren der Ellbogenschiene an der Unterarmschiene (5) sowohl in deren Parkzustand als auch in deren Betriebszustand in einem geeigneten Anstellwinkel bezüglich der Unterarmschiene (5) und zum Lösen der jeweiligen Arretierung ausgebildet sind; und
- mindestens ein flexibles und in seinem Umfang individuell anpassbares Oberarmbefestigungsband, das zum Umlegen um den Oberarm zur Fixierung der Ellbogenschiede daran ausgebildet ist und insbesondere durch einen Klettverschluss oder einen andersartigen Verschluss verschließbar ausgebildet sein kann.

9. Modularer Bausatz (12) zum Aufbau einer Universalschiene (1) nach einem der vorhergehenden Ansprüche, umfassend:
- eine oder mehrere unterschiedlich dimensionierte und/oder geformte Basisplatte(n) (3) mit mindestens einem flexiblen und in seinem Umfang individuell anpassbaren Handbefestigungsband (14), das zum Umlegen um die Hand zur Fixierung der Basisplatte (3) daran ausgebildet ist;
- eine oder mehrere unterschiedlich dimensionierte und/oder geformte, jeweils lösbar an der Basisplatte (3) befestigbare Unterarmschiene(n) (5) mit jeweils mindestens einem flexiblen und in seinem Umfang individuell anpassbaren Unterarmbefestigungsband (15), das zum Umlegen um den Unterarm zur Fixierung der Unterarmschiene (5) daran ausgebildet ist; sowie
- für jeden Finger einer Hand mindestens ein oder mehrere unterschiedlich dimensionierte und/oder geformte, jeweils lösbar an der Basisplatte (3) befestigbare Fingerelement(e) (4) jeweils umfassend eine zum Abstützen jeweils eines ganzen Fingers länglich ausgebildete Fingerschiene (6) und mindestens ein flexibles und in seinem Umfang individuell anpassbares Fingerbefestigungsband (7), das zum Umlegen um den Finger zur Fixierung der Fingerschiene (6) daran ausgebildet ist, wobei jede Fingerschiene (6) für transversale Kraftbelastungen in einem ersten Kraftintervall, das mit einem menschlichen Finger aufbringbare Kräfte abdeckt, starr oder in vorbestimmten Grenzen elastisch biegsam ist und in einem höheren zweiten Kraftintervall derart plastisch verformbar ist, dass sie durch eine plastische Biegung durch Kräfte im zweiten Kraftintervall dauerhaft an eine individuelle Fingerform und - Ausrichtung anpassbar ist;
- wobei die eine oder die mehreren Basisplatten (3) eine zum Auflegen der Handfläche darauf glatt ausgebildete Oberseite (8) und eine von dieser abgewandten Unterseite (2), an der die Unterarmschiene (5) und das mindestens eine Fingerelement (4) lösbar befestigbar sind, aufweisen, wobei die Oberseite (8) der einen oder der mehreren Basisplatten (3) sich zusammensetzt aus einem ersten ebenen Flächenabschnitt (10), der zum Abstützen eines etwa bis zu den Fingergrundgelenken reichenden Großteils einer Handfläche ausgebildet ist, und einem zweiten ebenen Flächenabschnitt (11), der zum Abstützen der restlichen Handfläche etwa ab den Fingergrundgelenken ausgebildet ist; und wobei der erste Flächenabschnitt (10) und der zweite Flächenabschnitt (11) ohne Unterbrechung und unter Ausbildung eines geradlinigen oder gebogenen Knicks (9) in der Oberseite (8) der einen oder der mehreren Basisplatten (3) ineinander übergehen, sodass sie miteinander einen stumpfen Winkel zwischen 180° und 270° bilden, gemessen in einer sich von der Oberseite (8) der Basisplatte (3) nach oben erstreckenden normalen Halbebene, die sich rechtwinklig zum Knick (9) erstreckt.

10. Modularer Bausatz (12) nach Anspruch 9, ferner umfassend:
- ein Schränkeisen (16) zum formgebenden plastischen Biegen der Fingerschienen (6) und/oder der Unterarmschiene(n) (5) zu deren Anpassung an unterschiedliche Anwendungsfälle und Patienten;
- wobei das Schränkeisen (16) einen Satz von zwei oder mehr drehbar miteinander verbindbaren Konturiereisen (17) mit jeweils einem oder mehreren Vorsprüngen (18) aufweist, die derart ausgebildet sind, dass die zu biegende Finger- oder Unterarmschiene (6, 5) zwischen den Vorsprüngen (18) zweier verschränkter, drehbar miteinander verbundener Konturiereisen (17) einspannbar und durch das Drehen dieser Konturiereisen (17) relativ zueinander zu einer gewünschten Form verbiegbar ist.

11. Modularer Bausatz (12) nach Anspruch 9 oder 10, ferner umfassend:
- einen Überzug (13) aus weichem, insbesondere textilem, Material, der zum Aufsetzten und hüllenartigen Überziehen der miteinander verbundenen Basisplatte (3) und Unterarmschiene (5) ausgebildet ist;
- wobei die zugehörigen Hand- und Unterarmbefestigungsbänder (14, 15) im Überzug (13) integriert sind.

## Claims

1. Modifiable universal splint (1) for wrist and/or finger stabilization, comprising:
- a flat base plate (3) designed to be rigid for supporting a palm, with at least one flexible hand fastening strap (14) that can be individually adjusted in its circumference and is designed to be wrapped around the hand to fix the base plate (3) to it;
- an elongated forearm splint (5) that can be detachably attached to the base plate (3) and is rigidly designed to support a forearm, with at least one flexible forearm fastening strap (15) that can be individually adjusted in circumference and is designed to be wrapped around the forearm to secure the forearm splint (5) thereto; and
- at least one finger element (4) that can be detachably attached to the base plate (3), comprising an elongated finger splint (6) for supporting a whole finger and at least one flexible finger fastening strap (7) that can be individually adjusted in circumference and is designed to be wrapped around the finger to secure the finger splint (6) to it;
- wherein each finger splint (6) is rigid or elastically flexible within predetermined limits for transverse force loads in a first force interval covering forces that can be applied with a human finger, and is plastically deformable in a higher second force interval in such a way that it is permanently adaptable to an individual finger shape and orientation by plastic bending due to forces in the second force interval;
- the base plate (3) has a smooth upper side (8) for resting the palm of the hand on it and a lower side (2) facing away from it, to which the forearm splint (5) and the at least one finger element (4) are detachably attached;
- the upper side (8) of the base plate (3) consists of a first flat surface section (10), which is designed to support a large part of the palm of the hand extending approximately to the metacarpophalangeal joints, and a second flat surface section (11), which is designed to support the remaining part of the palm of the hand approximately from the metacarpophalangeal joints; and
- the first surface section (10) and the second surface section (11) merge without interruption and form a straight or curved bend (9) in the upper side (8) of the base plate (3), so that they form an obtuse angle between 180° and 270° with each other, measured in a normal half-plane extending upward from the upper side (8) of the base plate (3) and extending at right angles to the bend (9).

2. Universal splint (1) according to claim 1, wherein
- each finger splint (6) is formed from a wire bent into a double wire in an approximately triangular, rectangular or U-shape, or is formed as a double wire from two individual wires connected to each other;
- wherein the wire thickness corresponds to the said force intervals and the double wire is attached to the base plate (3) with the proximal ends of its two legs or single wires is attached to the base plate (3) so that its triangular, rectangular or U-shaped end or its connected distal single wire ends form a free distal length section of the finger splint (6).

3. Universal splint (1) according to claim 2, wherein
- the two legs or single wires of the double wire run approximately parallel to each other at a distance of slightly less than a finger width apart, such that they support a finger resting on the finger splint (6) along its longitudinal axis on both sides thereof; and/or
- the double wire is plastically bent at one or more points distributed along its length in order to adapt to an individual finger shape and orientation in a transverse direction that is perpendicular to a plane locally spanned by each of the two legs or individual wires.

4. Universal splint (1) according to one of the preceding claims, in which
- the forearm splint (5) covers transverse force loads in a third force interval, which is associated with a human forearm when wearing the universal splint (5), and is rigid or elastically flexible within predetermined limits, and is plastically deformable in a higher fourth force interval in such a way that it can be permanently adapted to an individual forearm shape and wrist position by plastic bending due to forces in the fourth force interval.

5. Universal splint (1) according to claim 4, in which
- the forearm splint (5) is formed from a wire bent into a double wire in an approximately triangular, rectangular, or U-shape, or is designed as a double wire consisting of two individual wires connected to each other;
- the wire thickness corresponds to the force intervals specified for the forearm splint, and the double wire of the forearm splint (5) is attached to the base plate (3) with the proximal ends of its two legs or single wires to the base plate (3) so that its triangular, rectangular or U-shaped end or its connected distal single wire ends form a free distal length section of the forearm splint (5).

6. Universal splint (1) according to claim 5, wherein
- the two legs or individual wires of the U-shaped double wire of the forearm splint (5) run approximately parallel to each other at a distance of slightly less than one forearm width from each other, such that they support a forearm resting on the forearm splint (5) along its longitudinal axis on both sides thereof; and/or
- the double wire of the forearm splint (5) is plastically bent at one or more points distributed along its length in order to adapt to an individual forearm shape and wrist position in a transverse direction that is perpendicular to a plane locally spanned by each of the two legs or individual wires of the forearm splint (5).

7. Universal splint (1) according to one of the preceding claims, in which
- the detachable connections of the forearm splint (5) and/or the finger elements (4) to the base plate are designed as screw connections or screw-clamp connections, preferably with length adjustability of the forearm splint (5) and/or the finger elements (4) by means of their displacement in their longitudinal direction along the respective screw connection or screw clamp connections; and/or
- the flexible hand, forearm, and/or finger fastening straps (14, 15, 7) are each designed to be closed with a fastener, in particular a Velcro fastener.

8. Universal splint (1) according to one of the preceding claims, further comprising
- an elbow splint, which is rigid and elongated for supporting the elbow and/or upper arm and is connected to the distal end of the forearm splint (5) facing away from the base plate (3) in such a way that it can be rotated from a parking position completely applied to the forearm splint (5) to a position away from the forearm splint (5) ; and
- one or more release and locking devices, which are designed to lock the elbow splint to the forearm splint (5) both in its parked state and in its operational state at a suitable angle of attack relative to the forearm splint (5) and to release the respective lock; and
- at least one flexible upper arm fastening strap, the circumference of which can be individually adjusted, which is designed to be wrapped around the upper arm to fix the elbow splint thereto and which can be closed in particular by means of a Velcro fastener or another type of fastener.

9. Modular kit (12) for constructing a universal splint (1) according to one of the preceding claims, comprising:
- one or more base plates (3) of different sizes and/or shapes, with at least one flexible hand strap (14) that can be individually adjusted in circumference and is designed to be wrapped around the hand to secure the base plate (3) to it;
- one or more differently dimensioned and/or shaped forearm splints (5), each of which can be detachably fastened to the base plate (3), each with at least one flexible forearm fastening strap (15) that can be individually adjusted in circumference and is designed to be wrapped around the forearm to secure the forearm splint (5) thereto; and
- for each finger of a hand, at least one or more finger elements (4) of different sizes and/or shapes, each of which can be detachably attached to the base plate (3), each comprising an elongated finger splint (6) for supporting an entire finger and at least one flexible finger fastening strap (7) that can be individually adjusted in circumference, which is designed to be wrapped around the finger to fix the finger splint (6) thereto, wherein each finger splint (6) being rigid or elastically flexible within predetermined limits for transverse force loads in a first force interval covering forces that can be applied with a human finger, and being plastically deformable in a higher second force interval in such a way that it can be permanently adapted to an individual finger shape and orientation by plastic bending due to forces in the second force interval;
- wherein the one or more base plates (3) have an upper side (8) that is smooth for resting the palm of the hand on it and a lower side (2) facing away from it, to which the forearm splint (5) and the at least one finger element (4) can be detachably attached, wherein the upper side (8) of the one or more base plates (3) comprises a first flat surface section (10) designed to support a large part of a palm extending approximately to the metacarpal joints, and a second flat surface section (11) designed to support the remaining palm of the hand approximately from the metacarpophalangeal joints; and wherein the first surface section (10) and the second surface section (11) merge into one another without interruption and forming a straight or curved bend (9) in the upper side (8) of the one or more base plates (3), so that they form an obtuse angle between 180° and 270° with each other, measured in a normal half-plane extending upward from the upper surface (8) of the base plate (3) and extending at right angles to the bend (9).

10. Modular kit (12) according to claim 9, further comprising:
- a bending iron (16) for plastic bending of the finger splints (6) and/or the forearm splint(s) (5) to adapt them to different applications and patients;
- wherein the bending iron (16) comprises a set of two or more contouring irons (17) that can be rotatably connected to each other, each with one or more projections (18) that are designed such that the finger or forearm splint (6, 5) to be bent (6, 5) can be clamped between the projections (18) of two interlocking contouring irons (17) connected to each other in a rotatable manner and bent into a desired shape by rotating these contouring irons (17) relative to each other.

11. Modular kit (12) according to claim 9 or 10, further comprising:
- a cover (13) made of soft, in particular textile, material, which is designed to be placed over and cover the interconnected base plate (3) and forearm splint (5) like a sleeve;
- wherein the associated hand and forearm fastening straps (14, 15) are integrated into the cover (13).

## Revendications

1. Attelle universelle modifiable (1) pour la stabilisation du poignet et/ou des doigts, comprenant :
- une plaque de base (3) plate et rigide destinée à soutenir la paume de la main, avec au moins une sangle de fixation (14) flexible et ajustable individuellement sur son pourtour, conçue pour être enroulée autour de la main afin d'y fixer la plaque de base (3) ;
- une attelle d'avant-bras allongée (5) pouvant être fixée de manière amovible à la plaque de base (3), conçue de manière rigide pour soutenir un avant-bras, avec au moins une sangle de fixation d'avant-bras (15) flexible et ajustable individuellement dans sa circonférence, qui est conçue pour être enroulée autour de l'avant-bras afin d'y fixer l'attelle d'avant-bras (5) ; ainsi que
- au moins un élément pour doigt (4) pouvant être fixé de manière amovible à la plaque de base (3), comprenant une attelle pour doigt (6) de forme allongée pour soutenir un doigt entier et au moins une sangle de fixation pour doigt (7) flexible et ajustable individuellement dans sa circonférence, qui est conçue pour être enroulée autour du doigt afin d'y fixer l'attelle pour doigt (6) ;
- chaque attelle pour doigt (6) est rigide ou élastiquement flexible dans des limites prédéterminées pour les forces transversales comprises dans une première intervalle de forces couvrant les forces pouvant être exercées par un doigt humain, et est plastiquement déformable dans une deuxième intervalle de forces plus élevée, de telle sorte qu'elle peut être adaptée de manière permanente à la forme et à l'orientation individuelles du doigt par une flexion plastique sous l'effet des forces comprises dans la deuxième intervalle de forces ;
- la plaque de base (3) présente une face supérieure (8) lisse destinée à recevoir la paume de la main et une face inférieure (2) opposée à celle-ci, sur laquelle l'attelle d'avant-bras (5) et au moins un élément de doigt (4) sont fixés de manière amovible ;
- la face supérieure (8) de la plaque de base (3) se compose d'une première partie plane (10), qui est conçue pour soutenir une grande partie de la paume de la main s'étendant approximativement jusqu'aux articulations métacarpophalangiennes, et d'une deuxième partie plane (11), qui est conçue pour soutenir le reste de la paume de la main à partir approximativement des articulations métacarpophalangiennes ; et
- la première partie plane (10) et la deuxième partie plane (11) se rejoignent sans interruption et en formant un coude rectiligne ou courbé (9) dans la face supérieure (8) de la plaque de base (3), de manière à former entre elles un angle obtus compris entre 180° et 270°, mesuré dans un demi-plan normal s'étendant vers le haut à partir de la face supérieure (8) de la plaque de base (3) et s'étendant perpendiculairement au coude (9).

2. Attelle universelle (1) selon la revendication 1, dans laquelle
- chaque attelle pour doigt (6) est formée d'un fil métallique courbé en forme de triangle, de rectangle ou de U pour former un fil double, ou est conçue comme un fil double composé de deux fils individuels reliés entre eux ;
- l'épaisseur du fil correspondant aux intervalles de force mentionnés et le fil double étant fixé à la plaque de base (3) par les extrémités proximales de ses deux branches ou à la plaque de base (3), de sorte que son extrémité triangulaire, rectangulaire ou en forme de U ou ses extrémités distales reliées entre elles forment une partie distale libre de l'attelle (6).

3. Attelle universelle (1) selon la revendication 2, dans laquelle
- les deux branches ou fils individuels du fil double s'étendent de manière approximativement parallèle l'un à l'autre à une distance légèrement inférieure à la largeur d'un doigt, de telle sorte qu'ils soutiennent un doigt reposant sur l'attelle (6) le long de son axe longitudinal des deux côtés de celle-ci ; et/ou
- le fil double est plié plastiquement en un ou plusieurs endroits répartis sur sa longueur afin de s'adapter à la forme et à l'orientation individuelles du doigt dans une direction transversale perpendiculaire à l'un des deux bras ou fils individuels tendus localement.

4. Attelle universelle (1) selon l'une des revendications précédentes, dans laquelle
- l'attelle d'avant-bras (5) couvre les forces transversales dans un troisième intervalle de force, qui est rigide ou élastiquement flexible dans des limites prédéterminées, et dans un quatrième intervalle de force plus élevé, de telle sorte qu'elle peut être déformée plastiquement par une force pouvant être appliquée sur un avant-bras humain lorsqu'il porte l'attelle universelle (5), et dans un quatrième intervalle de force supérieur, il est déformable plastiquement de telle sorte qu'il peut être adapté de manière permanente à la forme individuelle de l'avant-bras et à la position du poignet par une déformation plastique due aux forces dans le quatrième intervalle de force.

5. Attelle universelle (1) selon la revendication 4, dans laquelle
- l'attelle d'avant-bras (5) est formée d'un fil métallique courbé en forme de triangle, de rectangle ou de U pour former un fil double, ou est conçue comme un fil double composé de deux fils individuels reliés entre eux ;
- l'épaisseur du fil correspondant aux intervalles de force indiqués pour l'attelle d'avant-bras et le fil double de l'attelle d'avant-bras (5) étant fixé à la plaque de base (3) par les extrémités proximales de ses deux branches ou des fils individuels à la plaque de base (3), de sorte que son extrémité triangulaire, rectangulaire ou en forme de U ou ses extrémités distales de fils individuels reliées entre elles forment une partie distale libre de l'attelle d'avant-bras (5).

6. Attelle universelle (1) selon la revendication 5, dans laquelle
- les deux branches ou fils individuels du fil double en forme de U de l'attelle d'avant-bras (5) s'étendent de manière approximativement parallèle l'un à l'autre à une distance légèrement inférieure à la largeur d'un avant-bras, de telle sorte qu'ils soutiennent un avant-bras reposant sur l'attelle d'avant-bras (5) le long de son axe longitudinal des deux côtés de celui-ci ; et/ou
- le fil double de l'attelle d'avant-bras (5) est plié de manière plastique en un ou plusieurs endroits répartis sur sa longueur afin de s'adapter à la forme individuelle de l'avant-bras et à la position du poignet dans une direction transversale perpendiculaire à un plan localement défini par chacune des deux branches ou fils individuels de l'attelle d'avant-bras (5).

7. Attelle universelle (1) selon l'une des revendications précédentes, dans laquelle
- les liaisons amovibles de l'attelle d'avant-bras (5) et/ou des éléments pour doigt (4) avec la plaque de base sont réalisées sous forme de liaisons vissées ou de liaisons vissées-serrées, de préférence avec un réglage en longueur de l'attelle d'avant-bras (5) et/ou des éléments pour doigt (4) par leur déplacement dans leur direction longitudinale le long de la liaison vissée ou des liaisons vissées-serrées respectives ; et/ou
- les sangles de fixation flexibles pour la main, l'avant-bras et/ou les doigts (14, 15, 7) sont chacune conçues pour être fermées à l'aide d'une fermeture, en particulier une fermeture velcro.

8. Attelle universelle (1) selon l'une des revendications précédentes, comprenant en outre
- une attelle de coude, qui est rigide et allongée pour soutenir le coude et/ou le bras et qui est reliée de manière pivotante à l'extrémité distale de l'attelle d'avant-bras (5) opposée à la plaque de base (3) de telle sorte qu'elle peut être déployée à partir d'une position de repos complètement appliquée contre l'attelle d'avant-bras (5) dans une position de fonctionnement ; ainsi que
- un ou plusieurs dispositifs de déverrouillage et de blocage, qui sont conçus pour bloquer l'attelle de coude sur l'attelle d'avant-bras (5) aussi bien dans sa position de repos que dans sa position de fonctionnement, selon un angle d'attaque approprié par rapport à l'attelle d'avant-bras (5), et pour déverrouiller le blocage correspondant ; et
- au moins une sangle de fixation flexible et ajustable individuellement dans sa circonférence, qui est conçue pour être placée autour du bras afin d'y fixer l'attelle de coude et qui peut notamment être fermée par une fermeture velcro ou un autre type de fermeture.

9. Kit modulaire (12) pour la construction d'une attelle universelle (1) selon l'une des revendications précédentes, comprenant :
- une ou plusieurs plaques de base (3) de dimensions et/ou de formes différentes, avec au moins une sangle de fixation pour la main (14) flexible et ajustable individuellement dans sa circonférence, qui est conçue pour être enroulée autour de la main afin d'y fixer la plaque de base (3) ;
- une ou plusieurs attelles d'avant-bras (5) de dimensions et/ou de formes différentes, pouvant être fixées de manière amovible à la plaque de base (3), chacune comportant au moins une sangle de fixation d'avant-bras (15) flexible et ajustable individuellement dans sa circonférence, qui est conçue pour être enroulée autour de l'avant-bras afin d'y fixer l'attelle d'avant-bras (5) ; et
- pour chaque doigt d'une main, au moins un ou plusieurs éléments pour doigts (4) de dimensions et/ou de formes différentes, pouvant être fixés de manière amovible à la plaque de base (3), comprenant chacun une attelle pour doigt (6) de forme allongée destinée à soutenir un doigt entier et au moins une sangle de fixation pour doigt (7) flexible et ajustable individuellement dans sa circonférence, qui est conçu pour être placé autour du doigt afin d'y fixer l'attelle (6), chaque attelle (6) étant rigide ou élastiquement flexible dans des limites prédéterminées pour les contraintes transversales comprises dans une première intervalle de forces couvrant les forces pouvant être exercées par un doigt humain, et étant déformable plastiquement dans une deuxième intervalle de forces plus élevée, de telle sorte qu'elle peut être adaptée de manière permanente à la forme et à l'orientation individuelles du doigt par une flexion plastique sous l'effet des forces comprises dans la deuxième intervalle de forces ;
- la ou les plaques de base (3) présentant une face supérieure (8) lisse pour y poser la paume de la main et une face inférieure (2) opposée à celle-ci, sur laquelle l'attelle d'avant-bras (5) et le ou les éléments pour doigt (4) peuvent être fixés de manière amovible, la face supérieure (8) de la ou des plaques de base (3) se composant d'une première partie plane (10) conçue pour soutenir une grande partie de la paume de la main s'étendant approximativement jusqu'aux articulations métacarpophalangiennes, et d'une deuxième partie plane (11) conçue pour soutenir le reste de la paume à partir des articulations métacarpophalangiennes ; et la première partie (10) et la deuxième partie (11) se rejoignant sans interruption et en formant un pli rectiligne ou courbé (9) dans la face supérieure (8) de la ou des plaques de base (3), de manière à former entre elles un angle obtus compris entre 180° et 270°, mesuré dans un demi-plan normal s'étendant vers le haut à partir de la face supérieure (8) de la plaque de base (3) et s'étendant perpendiculairement au coude (9).

10. Kit modulaire (12) selon la revendication 9, comprenant en outre :
- une ferrure de renfort (16) pour le pliage plastique des attelles pour doigts (6) et/ou de la ou des attelles pour avant-bras (5) afin de les adapter à différents cas d'application et patients ;
- l'outil de formage (16) comportant un ensemble de deux ou plusieurs outils de profilage (17) pouvant être reliés entre eux de manière pivotante, chacun comportant une ou plusieurs saillies (18) qui sont conçues de telle sorte que l'attelle pour doigts ou avant-bras (6, 5) à plier (6, 5) à plier puisse être serrée entre les saillies (18) de deux fers de profilage (17) entrelacés et reliés entre eux de manière pivotante, et puisse être pliée à la forme souhaitée par la rotation de ces fers de profilage (17) l'un par rapport à l'autre.

11. Kit modulaire (12) selon la revendication 9 ou 10, comprenant en outre :
- une housse (13) en matériau souple, en particulier textile, qui est conçue pour être placée et enfilée comme une enveloppe sur la plaque de base (3) et l'attelle d'avant-bras (5) reliées entre elles ;
- les sangles de fixation pour la main et l'avant-bras (14, 15) correspondantes étant intégrées dans la housse (13).
